Europäisches Patentamt

European Patent Office

Office européen des brevets

(11) Veröffentlichungsnummer: **0 237 039 B1**

## EUROPÄISCHE PATENTSCHRIFT

(45) Veröffentlichungstag der Patentschrift: **01.07.92**

(21) Anmeldenummer: **87103458.3**

(22) Anmeldetag: **10.03.87**

(51) Int. Cl.5: **C12M 1/10**, C12M 1/02, C12M 1/04, C12M 1/00, B01D 19/00, C02F 3/28

(54) **Verfahren zur Durchführung gasbildender biotechnologischer Prozesse in Festbettreaktoren und zum Be- und Entgasen geeignete Vorrichtungen.**

(30) Priorität: **14.03.86 DE 3608466**
**20.09.86 DE 3632093**

(43) Veröffentlichungstag der Anmeldung:
**16.09.87 Patentblatt 87/38**

(45) Bekanntmachung des Hinweises auf die Patenterteilung:
**01.07.92 Patentblatt 92/27**

(84) Benannte Vertragsstaaten:
**AT BE CH DE ES FR GB GR IT LI LU NL SE**

(56) Entgegenhaltungen:
**WO-A-86/03481**
**FR-A- 1 329 738**
**FR-A- 2 528 030**
**JP-A- 5 955 184**

**PATENT ABSTRACTS OF JAPAN, Band 8, Nr. 150 (C-233) (1587), 12. Juli 1984**

(73) Patentinhaber: **Forschungszentrum Jülich GmbH**
**Postfach 1913**
**W-5170 Jülich(DE)**

(72) Erfinder: **Overath, Horst**
**Bastionstr. 9**
**W-5170 Jülich(DE)**
Erfinder: **Soeder, Carl-Johannes, Prof.**
**Diemelstr. 5**
**W-4600 Dortmund 41(DE)**
Erfinder: **Salhani, Nazir, Dr.**
**Pfalzgrafenstr. 2**
**W-5100 Aachen(DE)**

EP 0 237 039 B1

## Beschreibung

Die Erfindung bezieht sich auf ein Verfahren zur Durchführung von unter Gasbildung ablaufenden biotechnologischen Prozessen in Reaktoren mit einem horizontalen Schüttkörperbett aus von Mikroorganismen bewachsenen Trägerkörpern, sowie auf Vorrichtungen zur Durchführung des Verfahrens in einem Festbett-Rohrreaktor mit Flüssigkeitszu-und -ableitung und Mitteln zur Gasabnahme und ggf. -zuführung.

Zahlreiche biotechnologische Prozesse laufen unter Freisetzung von Gas ab. So entsteht bei anaeroben Abbauprozessen Biogas ($CH_4$) und bei aeroben Gärprozessen $CO_2$. Ein weiteres Beispiel für unter Gasentwicklung ablaufende Bioreaktionen ist die bakterielle Denitrifikation, bei der Nitrat in Anwesenheit geeigneter Bakterien sowie organischer Substanzen als indirekte Reduktionsmittel unter sauerstoffarmen oder -freien Reaktionsbedingungen abgebaut wird.

Für die Durchführung dieser Umsetzung werden vielfach säulenförmige Festbettreaktoren angewandt, mit bakterienbewachsenen Trägerkörpern, an denen die bakterielle Umsetzung erfolgt. Beim Betrieb dieser Reaktoren treten nun insbesondere bei zunehmender Reaktorgröße Probleme durch die mit der Bioreaktion einhergehende Gasentwicklung auf: Das entstehende Gas wird von den bewachsenen Körpern nur verzögert abgelöst und kann mit dem Fortgang der Reaktion zur teilweisen Blockade der mit Bakterien besetzten aktiven Oberfläche führen und zur Erhöhung des Filtrationswiderstandes, d.h. der Durchsatz ist weder optimal, noch erfolgt er mit konstanter Rate.

Näher untersucht wurden diese Phänomene bei der Denitrifikation in Festbettreaktoren, bei denen das organische Substrat entweder dem zufließenden nitrathaltigen Wasser zudosiert oder aus Kunststoff-Füllkörpern durch Diffusion zur Verfügung gestellt wurde. Bei der bakteriellen Umsetzung wird $N_2$ entwickelt, dessen Ablösung aus der bakterienbesetzten Füllkörperschicht Probleme bereitet.

Der Erfindung liegt daher die Aufgabe zugrunde, ein Verfahren zur Intensivierung der Gasablösung und damit Verbesserung der bakteriellen Umsetzung vorzusehen.

Das zu diesem Zweck entwickelte erfindungsgemäße Verfahren ist dadurch gekennzeichnet, daß man den Reaktor ständig oder intermittierend mit Taktzeiten von 0 bis 25 min in Dreh- oder Schaukelbewegungen um seine Längsachse mit $\leq$ 10 Upm versetzt, die eine den Zusammenschluß und die Ablösung von Gasblasen vom Festkörper begünstigende Umwälzung des den Reaktor bis auf ein Restvolumen von maximal 35 % ausfüllenden biomasse-bewachsenen Schüttkörpers herbeiführen.

Vorzugsweise wird das Verfahren bei einer bakteriellen Denitrifikation angewandt, insb. als (Nitrit-Abbau)Stufe mit einer vorgeschalteten Nitrat → Nitrit-Abbaustufe.

Eine dafür geeignete Vorrichtung wird durch einen horizontalen Rohrreaktor gebildet mit einem den Reaktor weitgehend ausfüllenden Schüttkörperbett aus Trägerkörpern für Mikroorganismen und mit Flüssigkeitszu- und ableitung sowie Mitteln zur Gasabnahme und ggf. -zuführung, der im wesentlichen gekennzeichnet ist durch eine Lagerung und Antriesbmittel für die kontinuierliche oder intermittierende Rotation oder Schaukelbewegung des Reaktors mit maximal 10 Upm um seine Achse sowie einen Freiraum von maximal 35 % über der Schüttung, der eine Umwälzung derselben bei Rotation zuläßt.

Vorzugsweise ist die Längsachse um zumindest 3°, insb. etwa 5 - 10°, gegen die Horizontale geneigt.

Im Schüttkörperbett ist insb. ein Freiraum von 10 - 35 %, speziell 15 - 20 % über dem Schüttkörper vorhanden.

Die Rotation oder Schaukelbewegung (rechts- und linkssinnig um einen Winkel von z.B. 350° entsprechend der Bewegung von Waschmaschinentrommeln) kann kontinuierlich oder intermittierend erfolgen mit angemessenen Pausen. Zweckmäßig sind Taktzeiten von 5 - 25 Minuten und Drehgeschwindigkeiten von 0,2 - 10 Upm. Achs- oder Reibradantrieb (am Rohrmantel) sind zweckmäßig.

Als Anordnungen zur Gasabgabe eignen sich insb. eine oder mehrere Gasabgabe-Öffnung(en) am höher gelegenen Ende des Rohrmantels eines gegen die Horizontale geneigten Rohrreaktors mit Ventilelementen (insb. Kegelventilen mit Schließfeder) zum Verschließen der Öffnung(en) in Kombination mit Einrichtungen, wie insb. einen Nockenmechanismus, zur Freigabe dieser Öffnungen beim Passieren des höchsten Punktes.

Der drehbare Rohrreaktor hat vorzugsweise axiale Rohre oder Hohlachsen für den Flüssigkeitszu- und -ablauf mit so weit nach oben führendem Ablauf, daß der Flüssigkeitspegel im Drehrohr den Gassammelraum begrenzt. Zur Gasabführung kann die im Reaktor bis zum Flüssigkeitsspiegel hochgezogene Flüssigkeitsablaufleitung dienen, über die Gas und Flüssigkeit gemeinsam abgehen oder ein durch die Hohlachse geführtes Gasableitungsrohr, das im Rohrreaktor hinter einer abteilenden stirnseitigen Siebplatte nach oben gezogen ist.

In einem solchen Reaktor werden die von Mikroorganismen bewachsenen Füllkörper des einen Freiraum lassenden Festbetts durch langsame Drehung so bewegt, daß ein längeres Anhaften von Gasblasen unterbleibt und das Gas sich am höchsten Punkt sammelt und von dort kontinuierlich oder schubweise abgeführt werden kann.

Vorzugsweise sind achsparallele Rohre mit Öffnungen, Schlitzen oder Düsen längs der Reaktorinnenwand angeordnet und durch rippenartige Vorsprünge (mit Schlitzen) geschützt. Über solche Rohre, die auch hinter einem Siebmantel vor der Trägerkörperschüttung geschützt angeordnet sein können, kann die zu behandelnde Flüssigkeit über die Mantelfläche verteilt zugeführt werden. Gemäß einer besonders zweckmäßigen Anwendungsvariante dient jedoch ein solcher, mit Mantelrohren versehener Drehrohrreaktor zur Durchführung gasverbrauchender biotechnologischer Prozesse mit Sauerstoffverbrauch, wie z.B. für eine klassische Essigsäurefabrikation aus Äthanol, die Herstellung von Aminosäuren mit Corynebacterium glutamicum und zur aeroben Abwasserbehandlung. Es hat sich überraschenderweise gezeigt, daß durch eine solche Sauerstoffzufuhr über Mantelrohre im Drehrohrreaktor ein besonders intensiver $O_2$-Eintrag erreicht werden kann, der mit anderen Anordnungen zum effizienten Einrühren oder Zumischen von Sauerstoff bekannter Art nicht erreichbar ist.

Über die Hohlachse am tiefer gelegenen Ende tritt die mittels Pumpe oder über eine geeignete Niveaudifferenz geförderte Flüssigkeit (insb. über ein Sieb) in das Festbett ein, in dem die mikrobielle Stoffumsetzung erfolgt. Das Festbett befindet sich dank der Rotation des zylinderförmigen Reaktors in ständiger oder intermittierender Umwälzung, die durch Längsrippen an der Innenwand des Reaktors, die diesem eine erhöhte Steifigkeit verleihen, schlupffrei unterstützt werden kann.

Nach einer durchschnittlichen Verweildauer, die dem gewünschten Umsetzungsgrad bzw. der Reinigungsleistung angepaßt ist, tritt die behandelte Flüssigkeit durch die zweite Hohlachse über ein Sieb aus dem Reaktor aus.

Beide Hohlachsen sind insb. starr und über wasserdichte Lager mit dem Drehrohr verbunden und werden durch die Zu- bzw. Ableitung der Flüssigkeit verlängert. Von diesen reicht insbesondere der Ablauf bis zu einer Höhe, die für die Einstellung des Flüssigkeitspegels im Rohrreaktor unter Freilassung eines Restvolumens, in dem sich das gebildete Gas sammelt, ausreicht.

Gemäß einer Ausführungsvariante ist das geneigte, füllkörperhaltige Drehrohr mit einem Kranz von Gasabgabeschlitzen oder -öffnungen am höher gelegenen Ende versehen, die insb. über etwa 5 - 10 % der Bettlänge reichen und Mantellinien folgen, und es ist in einem etwas größeren feststehenden Zylindermantel stirnseitig gelagert, der einen Gasabgabestutzen am oberen Ende aufweist sowie an der gleichen Stirnseite einen darunterliegenden Flüssigkeitsauslaß. Auf diese Weise "schwimmt" das den Schüttkörper aufweisende Drehrohr in einem Flüssigkeitsmantel, wodurch der Energieaufwand für die Rotation vermindert wird.

Weitere Besonderheiten der Erfindung gehen aus der nachfolgenden Beschreibung von Ausführungsbeispielen unter Bezugnahme auf die angefügten Zeichnungen hervor; es zeigen schematisch:

Figur 1    einen geneigten Drehrohrreaktor mit hochgezogenem Gasableitungsrohr innerhalb des Drehbehälters hinter einer Siebplatte;

Figur 2    einen solchen Reaktor mit Gasabgabeöffnungen mit Ventilverschluß;

Figur 3    die Anordnung von Längsrohren an der Zylinderinnenwand in einem kleinen Ausschnitt und

Figur 4    einen "schwimmenden" Drehrohrreaktor, kombiniert mit einer Denitrifikationsvorstufe.

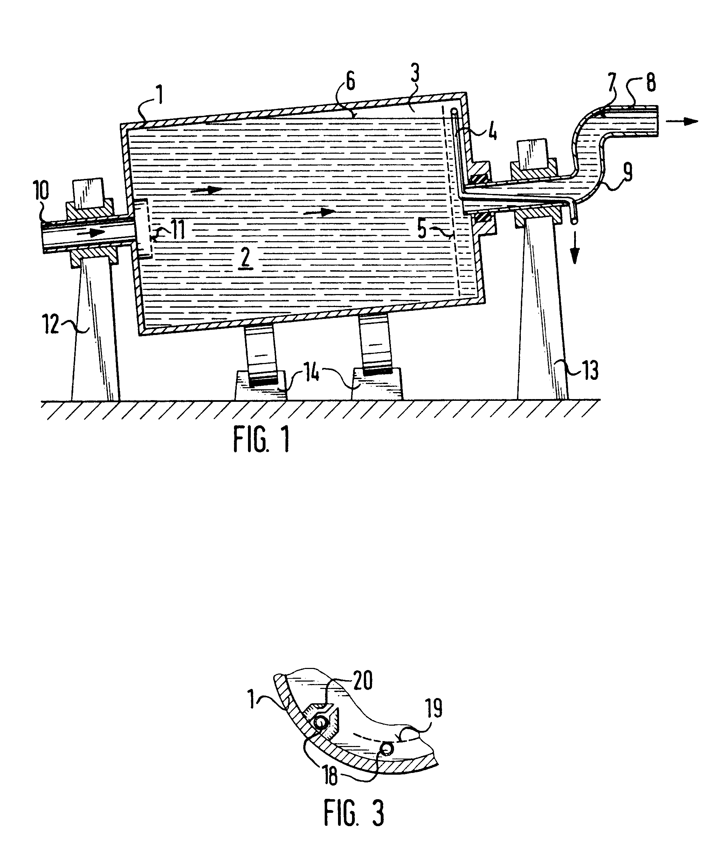

Gemäß Fig. 1 ist ein rohrförmiger Behälter 1 weitgehend durch Füllkörper 2 mit Mikrobenbewuchs gefüllt unter Freilassung eines Restvolumens 3, in dem sich das beim biologischen Prozeß gebildete Gas sammelt, das über ein Rohr 4 hinter einem Sieb 5 entweichen kann.

Durch die Linie 6 wird der Flüssigkeitsstand im geneigten Reaktor angedeutet, der dem Ablaufpegel 7 entspricht, der sich in der nach oben führenden Verlängerungsleitung 8 der ablaufseitigen Hohlachse 9 einstellt. Zulaufseitig ist analog eine Hohlachse 10 mit dem Reaktorrohr starr verbunden, die über eine (nicht gezeigte) Drehverbindung an eine Zulaufleitung anschließt.

Siebe 5 und 11 (ggf. mit einer gewissen Leitfunktion für die zutretende Flüssigkeit) schließen den Rohrraum zu den Hohlachsen hin ab, welch letztere in Lagerböcken 12, 13 gelagert sind. Durch 14 wird ein Stützlager mit Trommelantrieb angedeutet.

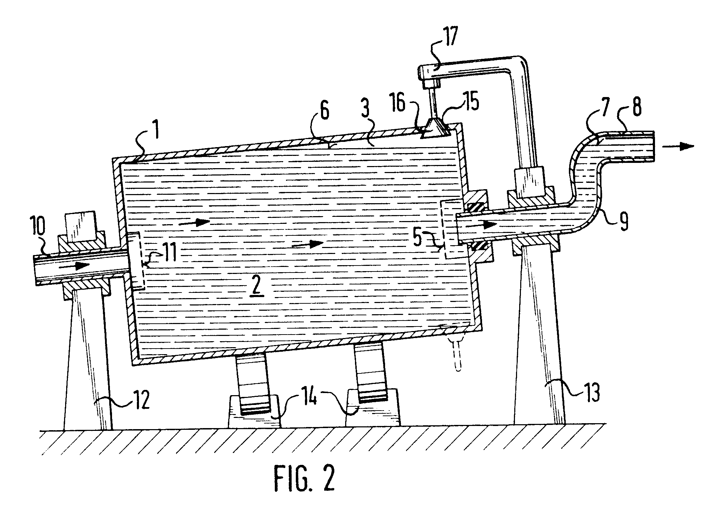

Die Anordnung gemäß Fig. 2 entspricht weitgehend derjenigen von Fig. 1, jedoch ist hier für die Gasabführung ein Kranz von Öffnungen 15 mit Ventilen 16 vorgesehen sowie ein Nockenmechanismus 17, bei dessen Passieren die federbelasteten Kegelventile geöffnet werden.

Der Drehrohrreaktor gem. Fig. 1 oder 2 kann an seiner Innenwand mit Längsrippen versehen sein, die für eine gewisse Mitnahme von Material bei der Drehbewegung sorgen. Diese Rippen sind insbesondere relativ flach, und ihre Reichweite beträgt vorzugsweise nicht mehr als 10 % des Reaktorradius.

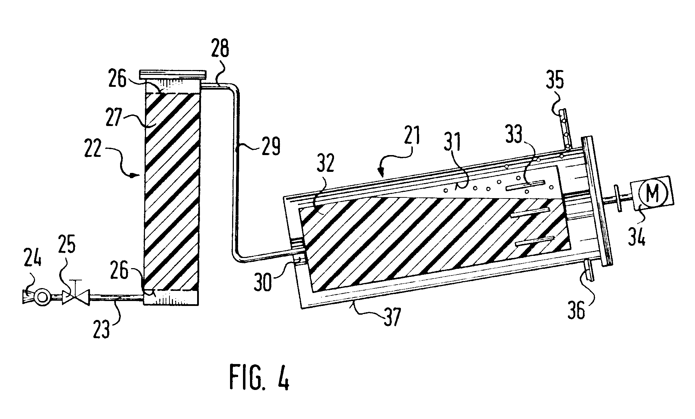

Gemäß einer bevorzugten Ausführungsart für größere Anlagen sind längs der Innenwand des Rohrreaktors Rohre 18 mit über ihre Länge verteilten Öffnungen für die Zuleitung von Flüssigkeit oder bei Durchführung von Begasungsreaktionen von Gas, insb. Luft, vorgesehen, die gegenüber der Schüttung etwa durch ein Stützsieb 19 parallel zur Reaktorwand oder durch geschlitzte Vorsprünge 20 abgeschirmt werden, wie es in Fig. 3 angedeutet ist.

Bei der insb. für kleinere Anlagen nützlichen Vorrichtung gem. Fig. 4 ist der geneigte Drehrohrreaktor 21 mit einem vorgeschalteten senkrechten Säulen-Reaktor 22 kombiniert, in den bei 23 kontinuierlich nitrathaltiges Wasser mittels einer Förderpumpe 24 über ein Regelventil 25 zur genauen Dosierung der Wasserdurchflußmenge eingeleitet wird. Der Säulenreaktor 22 enthält zwischen Siebplatten 26 als Füllkörper 27 z.B. ein inertes Kunststoffgranulat (z.B. Nitrex®-Filtermasse oder einen anderen geeigneten Füllkörper), das durch seine poröse oder rauhe Oberflächenstruktur günstige Besiedlungszonen für die Denitrifikationsbakterien bietet. Im Säulenreaktor 22 wird die erste Abbaustufe eingeleitet, bei der das Nitrat bakteriell weitgehend zu Nitrit reduziert bzw. das Wasser von Sauerstoff befreit wird.

Die Strömungsgeschwindigkeit des Wassers wird so geregelt, daß im Ablauf 28 des Säulenreaktors 22 ein hoher Nitritgehalt meßbar und im oberen Drittel des Reaktors keine Gasblasenbildung zu beobachten ist.

Über das Verbindungsrohr 29 wird das Wasser weiter zum Drehrohrreaktor 21 geleitet. Durch eine Hohlachse 30 mit Drehverbindung und Lagerung gelangt das Wasser in das drehbare Reaktor-Innenrohr 31, das zu etwa 3/4 mit einem geeigneten mit Denitrifikationsbakterien bewachsenen Granulat 32 gefüllt ist. Hier erfolgt anaerob der zweite Denitrifikationsschritt, wobei es zur Bildung von Stickstoffgas ($N_2$) und/oder von Lachgas ($N_2O$) kommt. Als Endstufe der Denitrifikation (Nitrat-Nitrit-Atmung) wird also hauptsächlich Stickstoff gasförmig entbunden und entweicht aus den im oberen Bereich des Innenrohres 31 angebrachten Entgasungsschlitzen 33 in die ohnehin zu etwa 80 Vol% Stickstoff enthaltende Luft.

Um das die Bakterienaktivität hemmende Anhaften von Gasblasen an der Oberfläche des Füllkörpers 32 zu vermeiden, wird das Innenrohr 31 alle 0 bis 15 Minuten mit Hilfe des Motors 34 drei Minuten lang mit 0,2 bis 3 Upm gedreht. Es ist nicht unbedingt erforderlich, das Drehrohr kontinuierlich um seine Längsachse zu bewegen. Sofern es nicht auf maximalen Durchsatz ankommt, wird das Innenrohr 31 beispielsweise alle 15 Minuten in Rotation versetzt. Die Gasabgabe wird gleichzeitig durch die schräge Lagerung des Reaktors in einem Winkel von 5 - 15° begünstigt. Über die Gasableitung 35 kann das Stickstoffgas endgültig die Apparatur verlassen. Das nitratarme Wasser kann dann über den Wasserauslauf 36 im Mantel 37 ebenfalls der Apparatur entzogen werden.

Beim Betrieb des Drehrohrreaktors ermöglicht die kontinuierliche Gasableitung aus den Porenräumen des Festbetts bzw. aus der Apparatur über die Entgasungsöffnungen ein ungestörtes Bakterienwachstum an der Matrixoberfläche. Ferner wird der ungestörte Wasserdurchfluß an den Oberflächen der Füllkörper, wo hauptsächlich die Abbauaktivität der Denitrifikationsbakterien stattfindet, gewährleistet. Die Bildung von Durchlaufrinnen innerhalb des Drehrohrreaktors, die den Kontakt zwischen Wasser und den Bakterien an der Matrix-Oberfläche behindern würden, findet hier nicht statt. Die weitgehende räumliche Trennung des Nitratabbaus in zwei Stufen mit Optimierung der Verweilzeiten in den Reaktoren 21 und 22, so daß im Säulenreaktor 22 bevorzugt lediglich der Nitratabbau zu Nitrit erfolgt (ohne Blasenbildung) bzw. der Sauerstoffgehalt auf Null reduziert wird, während im Drehrohrreaktor 21 dann der Abbau des Nitrits bis zur Stickstoffstufe bzw. zu $N_2$ stattfindet, ist technologisch besonders zweckmäßig.

Die bakterielle Denitrifikation im rotierenden geneigten bakterienbewachsenen Schüttkörperbett ist auch für die Regeneration von Wasser, insbesondere Meerwasser aus Aquarien oder aus Intensivhaltung von Wassertieren geeignet.

**Patentansprüche**

1. Verfahren zur Durchführung von unter Gasbildung ablaufenden biotechnologischen Prozessen in horizontalen Reaktoren mit einem Schüttkörperbett aus von Mikroorganismen bewachsenen Trägerkörpern, **dadurch gekennzeichnet,** daß man den Reaktor ständig oder intermittierend mit Taktzeiten von 0 bis 25 min in Dreh- oder Schaukelbewegungen um seine Längsachse mit ≤ 10 Upm versetzt, die eine den Zusammenschluß und die Ablösung von Gasblasen vom Festkörper begünstigende Umwälzung des den Reaktor bis auf ein Restvolumen von maximal 35 % ausfüllenden biomassebewachsenen Schüttkörpers herbeiführen.

2. Anwendung des Verfahrens nach Anspruch 1, bei der bakteriellen Denitrifikation insbesondere als zweite (Nitrit-Abbau)Stufe mit einer vorgeschalteten Nitrat → Nitrit-Abbaustufe.

3. Horizontaler Rohrreaktor zur Durchführung des Verfahrens nach Anspruch 1 mit einem den Reaktor (1) weitgehend ausfüllenden Schüttkörperbett aus Trägerkörpern für Mikroorganismen und mit Flüssigkeitszu- und ableitung sowie Mitteln zur Gasabnahme und ggf. -zuführung, **gekennzeichnet durch**

Lagerung (12, 13) und Antriebsmittel (14) für die kontinuierliche oder intermittierende Rotation oder Schaukelbewegung des Reaktors (1) mit maximal 10 Upm um seine Achse sowie einen Freiraum (3) von maximal 35 % über der Schüttung, der eine Umwälzung derselben bei Rotation zuläßt.

4. Reaktor nach Anspruch 3,
**dadurch gekennzeichnet,**
daß im Schüttkörperbett (1) ein Freiraum (3) von 10 - 35 %, insb. 15 - 20 % über dem Schüttkörper (2) vorhanden ist.

5. Reaktor nach Anspruch 3 oder 4,
**dadurch gekennzeichnet,**
daß die Drehgeschwindigkeit bei 0,2 - 10 Upm einstellbar ist.

6. Reaktor nach einem der Ansprüche 3 bis 5,
**dadurch gekennzeichnet,**
daß die Antriebsmittel (14) für eine taktweise intermittierende Rotation oder Schaukelbewegung, insb. mit einer Taktzeit von 5 - 25 min, geeignet sind.

7. Reaktor nach einem der Ansprüche 3 bis 6,
**gekennzeichnet durch**
axiale Rohre oder Hohlachsen (9, 10) für den Flüssigkeitszu- und -ablauf mit soweit nach oben führendem Ablauf (8), daß der Flüssigkeitspegel (6) im Drehrohr den Gassammelraum begrenzt.

8. Reaktor nach Anspruch 7,
**gekennzeichnet durch**
stirnseitige Siebe (11, 5) vor dem Anschuß der Hohlachse (9, 10).

9. Reaktor nach Anspruch 3 oder 6,
**gekennzeichnet durch**
Laufradantrieb und -abstützung (14).

10. Reaktor nach einem der Ansprüche 3 bis 9 ,
**gekennzeichnet durch**
nach innen vorspringenden Längsrippen des Rohrmantels.

11. Reaktor nach einem der Ansprüche 3 bis 10,
**gekennzeichnet durch**
achsparallele Zuführungsrohre (18) mit Öffnungen an der Innenwand des Rohrreaktors (1).

12. Reaktor nach Anspruch 11,
**dadurch gekennzeichnet,**
daß die Rohre (18) insb. in rippenartigen Vorsprüngen (20) geschützt sind.

13. Reaktor nach einem der Ansprüche 3 bis 12,
**gekennzeichnet durch**
eine um zumindest 3° gegen die Horizontale geneigte Achse.

14. Reaktor nach Anspruch 13,
**gekennzeichnet durch**
eine um etwa 10° gegen die Horizontale geneigte Achse.

15. Reaktor nach Anspruch 13 oder 14,
**gekennzeichnet durch**
eine oder mehrere Gasabgabe-Öffnung(en) (15) am höher gelegenen Ende des Rohrmantels (19) mit Ventil-Elementen (16) zum Verschließen der Öffnung(en) und durch Einrichtungen (17) zur Freigabe der Öffnungen bei deren Passieren des höchsten Punktes.

16. Reaktor nach Anspruch 13,
**dadurch gekennzeichnet,**
daß das Sieb (5) am höher gelegenen Ende über den gesamten Querschnitt reicht unter Abtrennung eines stirnseitigen schüttkörperfreien Raums, in dem die Flüssigkeitsableitung (9) selbst bis zum Flüssigkeitsspiegel hochgezogen oder ein durch diese aus dem Reaktor (1) herausgeführtes in den Gassammelraum (3) reichendes Gasableitungsrohr (4) angeordnet ist.

17. Reaktor nach Anspruch 16,
**dadurch gekennzeichnet,**
daß das Schüttkörperbett (31, 32) in einem Reaktormantel (37) mit Gas- und Flüssigkeitsauslässen (35, 36) "schwimmend" drehbar gelagert und mit Gasabgabeöffnungen (33) am "angehobenen Ende" versehen ist.

18. Reaktor nach Anspruch 17,
**dadurch gekennzeichnet,**
daß die Gasabgabeöffnungen durch einen Kranz von Öffnungen insb. Schlitzen (33) längs von Mantellinien gebildet wird.

19. Reaktor nach Anspruch 18,
**dadurch gekennzeichnet,**
daß die Schlitze (33) eine Länge von 5 - 10 % der Bettlänge insb. von 5 - 15 mm haben.

**Claims**

1. Method for carrying out biotechnology processes, the course of which is accompanied by gas formation, in horizontal reactors having a dumped or bulk material bed comprising carrier elements overgrown with micro-organisms, characterised in that the reactor is continually or intermittently with cycle times of 0 to 25 min given rotational or rocking movements about its longitudinal axis at ≤ 10 r.p.m., which brings about a circulation, promoting the combining and detaching of gas bubbles from the solid material, of the bulk material overgrown with biomass and filling the reactor apart from a residual volume of at most 35 %.

2. Use of the method according to claim 1 in bacterial denitrification especially as a second (nitrite decomposition) stage with a preceding nitrate → nitrite decomposition stage.

3. Horizontal tube reactor for carrying out the method according to claim 1, with a bulk material bed substantially filling the reactor (1) and comprising packing elements for microorganisms, and with liquid introduction and discharge means and also means for taking off and if appropriate introducing gas, characterised by bearing means (12, 13) and drive means (14) for the continuous or intermittent rotation or rocking of the reactor (1) at at most 10 r.p.m. about its axis, and also a free space (3) of at most 35 % above the bed, which free space allows circulation of the bed when rotation occurs.

4. Reactor according to claim 3, characterised in that a free space (3) of 10 - 35 %, especially 15 - 20 %, is present above the packing (2) in the packing element bed (1).

5. Reactor according to claim 3 or 4, characterised in that the rotational speed is settable at 0.2 - 10 r.p.m.

6. Reactor according to one of claims 3 to 5, characterised in that the drive means (14) are suitable for a rotational or rocking movement which is intermittent rhythmically, especially with a cycle time of 5 - 25 min.

7. Reactor according to one of claims 3 to 6, characterised by axial tubes or hollow shafts (9, 10) for liquid inflow and outflow, with an outlet (8) taken so high that the liquid level (6) in the rotary tube bounds the gas-collecting space.

8. Reactor according to claim 7, characterised by end screens (11, 5) before the connections of the hollowshafts (9, 10).

9. Reactor according to claim 3 or 6, characterised by running-wheel drive and support means (14).

10. Reactor according to one of claims 3 to 9, characterised by inwardly projecting longitudinal ribs of the tubular casing.

11. Reactor according to one of claims 3 to 10, characterised by axially parallel introduction pipes (18) with apertures on the inner wall of the tubular reactor (1).

12. Reactor according to claim 11, characterised in that the pipes (18) are protected especially in rib-like projections (20).

13. Reactor according to one of claims 3 to 12, characterised by an axis inclined through at least 3° relatively to the horizontal.

14. Reactor according to claim 13, characterised by an axis inclined by about 10° relatively to the horizontal.

15. Reactor according to claim 13 or 14, characterised by one or more gas discharge aperture or apertures (15) at the higher-situated end of the tubular casing (19) with valve elements (16) for closing the aperture(s) and by means (17) for opening the apertures as they pass the highest point.

16. Reactor according to claim 13, characterised in that the screen (5) at the higher-situated end extends over the entire cross-section, separating-off an end space which is free of packing elements and in which the liquid discharge conduit (9) itself is taken up to the liquid level, or a gas discharge pipe (4) is arranged which extends out of the reactor (1) through the said conduit and which extends into the gas-collecting space (3).

17. Reactor according to claim 16, characterised in that the packing element bed (31, 32) is mounted to be rotatable in "floating" manner in a reactor casing (37) having gas and liquid outlets (35, 36), and is provided with gas discharge apertures (33) at the "raised end".

18. Reactor according to claim 17, characterised in that the gas discharge apertures are formed by a circle of apertures especially slots (33) along generatrices.

19. Reactor according to claim 18, characterised in that the slots (33) have a length of 5 - 10 % of the bed length, especially of 5 - 15 mm.

**Revendications**

1. Procédé pour la mise en oeuvre d'opérations biotechnologigues s'effectuant avec formation de gaz, dans des réacteurs horizontaux comportant un lit de produit en vrac formé d'éléments porteurs recouverts de micro-organismes, caractérisé en ce qu'on soumet le réacteur à des mouvements rotatifs ou basculants autour de son axe longitudinal, de façon continue ou intermittente, à des durées de cycle de 0 à 25 minutes, à raison de 10 tours/minute ou moins, qui provoquent une circulation du produit en vrac recouvert de la biomasse et remplissant le réacteur de façon à laisser un volume résiduaire d'au plus 35 %, ladite circulation favorisant l'agglomération de bulles de gaz et leur séparation d'avec le corps solide.

2. Utilisation du procédé selon la revendication 1 pour la dénitrification bactérienne, en particulier comme deuxième étape (de décomposition de nitrites), comportant une étape préalable de décomposition du nitrate en nitrite.

3. Réacteur tubulaire horizontal pour la mise en oeuvre du procédé selon la revendication 1, comportant un lit de produit en vrac remplissant le réacteur dans une large mesure et formé d'éléments porteurs pour micro-organismes, et comportant des conduites d'amenée et d'évacuation de liquide, ainsi que des moyens pour l'évacuation et et éventuellement l'amenée de gaz, caractérisé par des paliers (12, 13) et un moyen d'entraînement (14) pour la rotation ou le mouvement basculant du réacteur (1) autour de son axe, de façon continue ou intermittente, à raison d'au plus 10 tr/min., ainsi qu'un espace libre (3) d'au plus 35 % au-dessus du produit en vrac, qui permet la circulation dudit produit pendant la rotation.

4. Réacteur selon la revendication 3, caractérisé en ce que le lit de produit en vrac (1) comprend un espace libre (3) de 10-35 %, en particulier 15-20 %, au-dessus du produit en vrac.

5. Réacteur selon la revendication 3 ou 4, caractérisé en ce qu'on peut régler la vitesse de rotation à 0,2-10 tr/min.

6. Réacteur selon une des revendications 3 à 5, caractérisé en ce que les moyens d'entraînement (14) sont appropriés pour un mouvement de rotation ou de bascule à cycles intermittents, en particulier à une durée de cycle de 5 - 25 minutes.

7. Réacteur selon une des revendications 3 à 6, caractérisé par des tubes axiaux ou des axes creux (9, 10), pour l'amenée et l'évacuation de liquide, avec un orifice de sortie (8) dirigé vers le haut de façon à ce que le niveau de liquide soit limité dans le tube rotatif de l'espace collecteur de gaz.

8. Réacteur selon la revendication 7, caractérisé par des tamis frontaux (11, 5) situés à l'avant du raccordement des axes creux (9, 10).

9. Réacteur selon la revendication 3 ou 6, caractérisé par une commande et un support (14) de roues mobiles.

10. Réacteur selon une des revendications 3 à 9, caractérisé par des nervures longitudinales de l'enveloppe de tube, faisant saillie vers l'intérieur.

11. Réacteur selon une des revendications 3 à 9, caractérisé par des tubes d'amenée (18) à axes parallèles, avec des ouvertures dans la paroi intérieure du réacteur tubulaire (1).

12. Réacteur selon la revendication 11, caractérisé en ce que les tubes (18) sont protégés, en particulier dans des saillies (20) du type nervures.

13. Réacteur selon une des revendications 3 à 12, caractérisé par un axe incliné d'au moins 3° par rapport à l'horizontale.

14. Réacteur selon la revendication 13, caractérisé par un axe incliné d'environ 10° par rapport à l'horizontale.

15. Réacteur selon la revendication 13 ou 14, caractérisé par une ou plusieurs ouvertures d'évacuation de gaz (15) à l'extrémité supérieure de l'enveloppe de tube (19), comportant des éléments soupapes (16) pour fermer les ouvertures, et par des dispositifs (17) pour ouvrir les ouvertures lorsqu'elles atteignent le point le plus élevé.

16. Réacteur selon la revendication 13, caractérisé en ce que le tamis (5) est disposé de façon à s'étendre sur toute la section transversale, jusqu'à l'extrémité supérieure, en formant une séparation pour un espace libre, du côté frontal, exempt de produit en vrac, dans lequel la conduite d'évacuation de produit (9) est prolongée elle-même vers le haut, jusqu'au niveau de liquide, ou par un tube d'évacuation de gaz (4) sortant du réacteur (1) en traversant ladite conduite d'évacuation et parvenant dans l'espace collecteur de gaz (3).

17. Réacteur selon la revendication 16, caractérisé en ce que le lit de produit en vrac (31, 32) est logé "flottant", de façon à pouvoir tourner dans une enveloppe de réacteur (37) comportant des sorties de gaz et de liquide (35, 36), et il est pourvu d'ouvertures (33) pour l'évacuation du gaz à l'extrémité supérieure.

18. Réacteur selon la revendication 17, caractérisé en ce que les ouvertures d'évacuation de gaz sont formées par une couronne d'ouvertures, en particulier de fentes, le long de génératrices.

19. Réacteur selon la revendication 18, caractérisé en ce que les fentes (33) ont une longueur qui vaut 5-10 % de la longueur du lit, en particulier de 5-15 mm.

FIG. 1

FIG. 3

EP 0 237 039 B1

FIG. 2

10

FIG. 4